# EUROPEAN PATENT APPLICATION

(11) **EP 4 603 493 A1**
(43) Date of publication of application: **20.08.2025**
(21) Application number: 23876729.7
(22) Date of filing: 11.10.2023
(51) Int. Cl.: C07F 9/6596, C07F 9/6564, C07F 9/6561, C07C 2/36

(54) **PHOSPHINE-PHENOL TRANSITION METAL COMPLEX, PREPARATION METHOD THEREFOR, AND USE THEREOF**

(30) Priority: 11.10.2022 CN 202211239447; 16.02.2023 CN 202310181067
(71) Applicant: CHINA PETROLEUM & CHEMICAL CORPORATION, Chaoyang District Beijing 100728 (CN); Sinopec (Beijing) Research Institute of Chemical Industry Co., Ltd., Beijing 100013 (CN)
(72) Inventor: GAO, Rong, Beijing 100013 (CN); GOU, Qingqiang, Beijing 100013 (CN); ZHANG, Randi, Beijing 100013 (CN); ZHENG, Gang, Beijing 100013 (CN); LAI, Jingjing, Beijing 100013 (CN); AN, Jingyan, Beijing 100013 (CN)
(74) Representative: Hoffmann Eitle
(86) International application number: PCT/CN2023/123999
(87) International publication number: WO 2024/078528

(57) **Abstract**

The present invention relates to a phosphine-phenol transition metal complex and preparation method therefor and use thereof. The structural formula of the phosphine-phenol transition metal complex is shown in formula (I), wherein, M is selected from Group IVB metals; Ar is selected from substituted or unsubstituted C6-C20 aryl; X is selected from halogen, C1-C10 hydrocarbyl, n is 1 or 2. The phosphine-phenol transition metal complex, when used as a primary catalyst of an olefin polymerization catalyst, can achieve higher activity in catalyzing olefin polymerization. The obtained polymer has a higher molecular weight, a narrower molecular weight distribution, and excellent copolymerization properties.

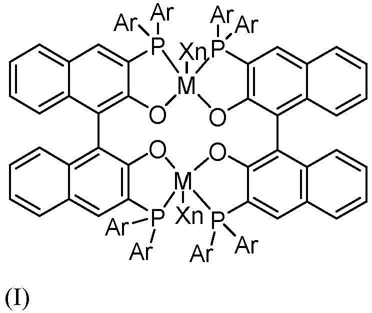

## Description

### Technical field

The present invention relates to the technical field of olefin polymerization catalysts, and particularly relates to a phosphine-phenol transition metal complex and preparation method therefor and use thereof.

### Background art

Polyolefin resins have excellent environmental coordination compared with other resin materials, and thus are widely used in industry and life. Polyethylene resin is an important polyolefin resin. Industrialized polyethylene catalysts include Ziegler-Natta type catalysts (see, for example, DE Pat 889229 (1953); IT Pat 545332 (1956) and IT Pat 536899 (1955); Chem. Rev., 2000, 100, 1169 and related literatures of the Special Edition), Phillips type catalysts (see, for example, Belg. Pat. 530617 (1955); Chem. Rev. 1996, 96, 3327) and metallocene catalysts (see, for example, W. Kaminsky, Metalorganic Catalysts for Synthesis and Polymerization, Berlin: Springer, 1999), as well as the late transition metal complexes-type highly efficient ethylene oligomerization and polymerization catalysts developed rapidly in recent years. In 2006, the Gibson group of British scientists discovered that Group IV phenol-phosphine zirconium complex has good catalytic ability for olefin polymerization, and can effectively carry out ethylene or propylene polymerization reaction under the activation of cocatalyst such as methylaluminoxane (MAO) (Inorg. chem, 2006, 45, 511-513, Organometallics 2008, 27, 235-245).

At present, there is an urgent need for high-performance olefin polymerization catalysts in the production of high-end polyolefins. Therefore, the development of new catalysts with good catalytic activity is of great significance.

### Contents of the invention

An object of the present invention is to provide a phosphine-phenol transition metal complex and preparation method therefor and use thereof, wherein the phosphine-phenol transition metal complex has excellent olefin homopolymerization/copolymerization performance, and the obtained polymer has a narrower molecular weight distribution.

In order to achieve the above object, in a first aspect, the present invention provides a phosphine-phenol transition metal complex, the structural formula of which is shown in formula (I), 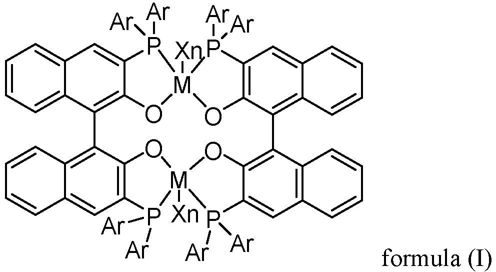 wherein, M is selected from Group IVB metals; Ar is selected from substituted or unsubstituted C6-C20 aryl; X is selected from halogen, C1-C10 hydrocarbyl, n is 1 or 2.

Preferably, C6-C20 aryl (including C6-C15 aryl) is selected from phenyl, 4-methyl phenyl, 4-ethyl phenyl, dimethyl phenyl, vinyl phenyl, anthryl, naphthyl, or biphenyl.

Preferably, for substituted C6-C20 aryl, the substituent thereof is selected from H, halogen, hydroxy, substituted or unsubstituted alkoxy (preferably C1-C6 alkoxy, more preferably methoxy, ethoxy or propoxy), substituted or unsubstituted C1-C20 hydrocarbyl.

Preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

Preferably, the halo is selected from fluoro, chloro, bromo, or iodo.

Preferably, the halo includes monohalo, dihalo or perhalo, such as monofluoro, difluoro or perfluoro.

Preferably, C1-C10 hydrocarbyl includes C1-C8 hydrocarbyl (such as C1-C6 alkyl) or C6-C10 aralkyl, the aralkyl including, but not limited to: phenylmethyl, phenylethyl, phenyl n-propyl, phenyl isopropyl, phenyl n-butyl and phenyl tert-butyl.

Preferably, C1-C20 hydrocarbyl includes C1-C8 hydrocarbyl (such as C1-C6 alkyl), preferably methyl, ethyl or propyl; or C6-C15 aryl, preferably phenyl.

Preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl.

Preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

Preferably, the halogen is selected from fluorine, chlorine, bromine and iodine.

Unless otherwise specified, the above definitions of groups also apply to the definitions of groups in other preferred or related structures described in the context.

Preferably, the structural formula of the phosphine-phenol transition metal complex is shown in formula (II), 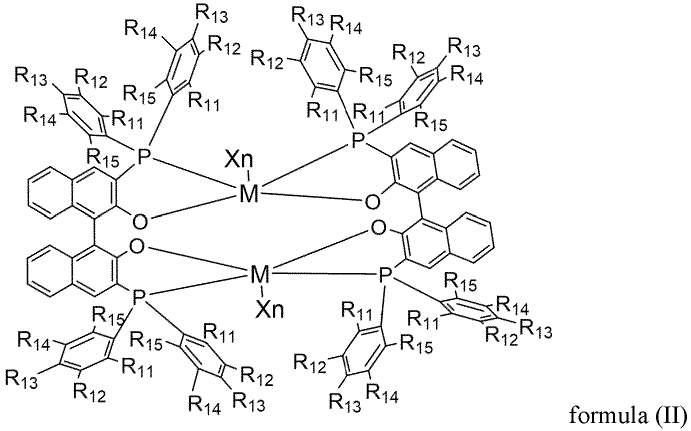 wherein, R₁₁-R₁₅ are each independently selected from H, halogen, hydroxy, substituted or unsubstituted alkoxy, substituted or unsubstituted C1-C20 hydrocarbyl; preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

Preferably, M is selected from titanium, zirconium and hafnium.

Preferably, R₁₁-R₁₅ are each independently selected from H, halogen, hydroxy, substituted or unsubstituted alkoxy, substituted or unsubstituted C1-C10 alkyl and substituted or unsubstituted C6-C15 aryl; X is selected from halogen, C1-C8 hydrocarbyl.

Preferably, C6-C15 aryl is selected from phenyl, 4-methyl phenyl, 4-ethyl phenyl, dimethyl phenyl, vinyl phenyl, anthryl, naphthyl, or biphenyl.

Preferably, for substituted alkoxy, substituted C1-C10 alkyl and substituted C6-C15 aryl, the substituents are independently selected from halogen, hydroxy, C1-C6 alkyl, halo-substituted C1-C6 alkyl, C1-C6 alkoxy and halo-substituted C1-C6 alkoxy.

Preferably, the halo is selected from fluoro, chloro, bromo, or iodo.

Preferably, the halo includes monohalo, dihalo or perhalo.

Preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl.

Preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

Preferably, the halogen is selected from fluorine, chlorine, bromine and iodine.

In a second aspect, the present invention provides a method for preparing the phosphine-phenol transition metal complex, the method comprising:
(1) reacting a compound of formula (III) with a compound of formula (IV), to obtain a ligand of formula (V);
(2) reacting the ligand with a hydrogen abstracting agent, and then reacting with an M metal compound, wherein, the transition metal M in the M metal compound is selected from Group IVB metals; 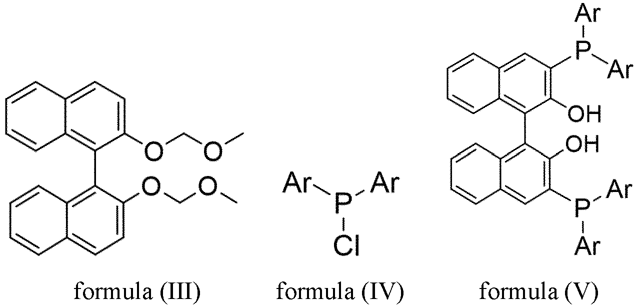 wherein, the definition of Ar is the same as described above.

Preferably, the M metal compound is selected from at least one of titanium tetrachloride, bis(tetrahydrofuran) titanium tetrachloride, tri(tetrahydrofuran) titanium trichloride, zirconium tetrachloride, bis(tetrahydrofuran) zirconium tetrachloride, hafnium tetrachloride, and bis(tetrahydrofuran) hafnium tetrachloride.

Preferably, the hydrogen abstracting agent is selected from at least one of NaH, KH, n-butyl lithium and methyl lithium.

In a third aspect, the present invention provides use of the phosphine-phenol transition metal complex in olefin polymerization.

In a fourth aspect, the present invention provides an olefin polymerization catalyst, comprising the above-mentioned phosphine-phenol transition metal complex and a cocatalyst.

Preferably, the cocatalyst is organoaluminum compound and/or organoboron compound.

Preferably, the organoaluminum compound is selected from one or more of alkylaluminoxane, alkyl aluminum and alkyl aluminum halide.

Preferably, the organoboron compound is selected from one or more of organoboron and organoborate.

In a fifth aspect, the present invention provides an olefin polymerization method, comprising carrying out olefin polymerization reaction in the presence of the olefin polymerization catalyst.

Preferably, the olefin polymerization reaction temperature is -78°C~200°C, preferably -20°C ~150°C, the pressure is 0.01~10MPa, preferably 0.01~5MPa.

The phosphine-phenol transition metal complex, when used as a primary catalyst of an olefin polymerization catalyst, can achieve high activity in catalyzing olefin polymerization. The obtained polymer has a higher molecular weight, a narrower molecular weight distribution, and excellent copolymerization properties.

Compared with the prior art, the technical solution of the present invention has the following advantages:
(1) The presently described synthesis method of the phosphine-phenol transition metal complex is simple and easy to perform.
(2) The presently described phosphine-phenol transition metal complex can catalyze the polymerization of olefins with high activity, especially maintaining a higher polymerization activity at a higher polymerization temperature.
(3) The presently described phosphine-phenol transition metal complex as a primary catalyst exhibits higher performance for copolymerization of ethylene with α-olefins or cycloolefins (such as norbomene).

### Description of figures

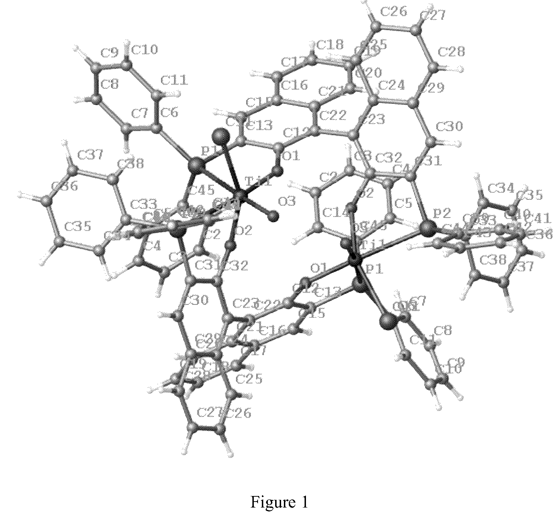

Figure 1 is a single crystal structural diagram of the phosphine-phenol transition metal complex prepared in Example 1.

### Detailed description of specific embodiments

The specific embodiments of the present invention are described in detail below. It should be understood that the specific embodiments described herein are only for illustrating and explaining the present invention, and are not intended to limit the present invention.

The endpoints of the ranges and any values disclosed herein are not limited to the exact ranges or values, and these ranges or values should be understood to encompass values close to these ranges or values. For numerical ranges, the value between the endpoint values of each range, the value between the endpoint values of each range and individual point values, and the value between individual point values can be combined with each other to obtain one or more new numerical ranges, which should be considered as having been specifically disclosed herein.

The structural formula of the phosphine-phenol transition metal complex according to the present invention is shown in formula (I), 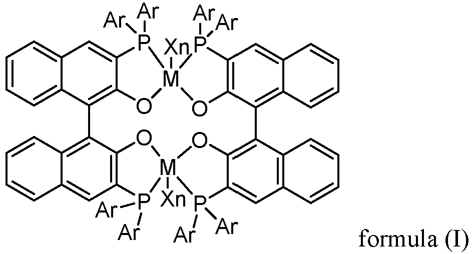 wherein, Ar is selected from substituted or unsubstituted C6-C20 aryl, preferably substituted or unsubstituted C6-C15 aryl, more preferably substituted or unsubstituted C6-C8 aryl.

Preferably, the structural formula of the phosphine-phenol transition metal complex is shown in formula (II), 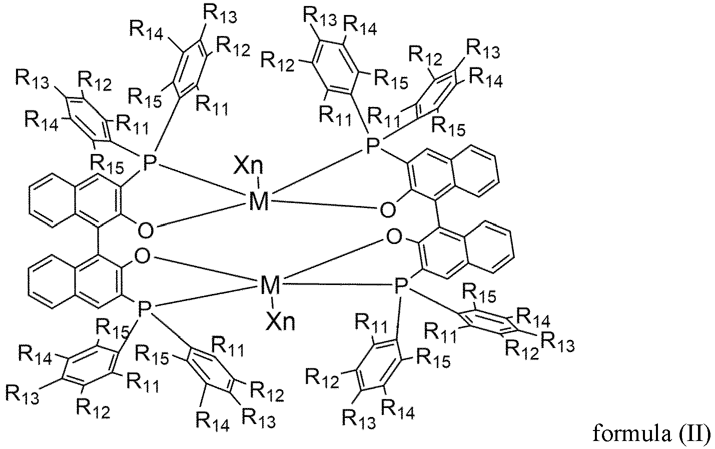 wherein, R₁₁-R₁₅ are each independently selected from H, halogen, hydroxy, substituted or unsubstituted alkoxy, substituted or unsubstituted C1-C20 hydrocarbyl. In a preferred case, R₁₁-R₁₅ are each independently selected from H, halogen, hydroxy, substituted or unsubstituted alkoxy, substituted or unsubstituted C1-C10 alkyl and substituted or unsubstituted C6-C15 aryl.

In formula (I) and formula (II), M is selected from Group IVB metals. In a preferred case, M is selected from titanium, zirconium and hafnium.

In formula (I) and formula (II), X is selected from halogen, C1-C10 hydrocarbyl. In a preferred case, X is selected from halogen, C1-C8 hydrocarbyl (such as C1-C6 alkyl).

In formula (I) and formula (II), n is 1 or 2, the number of X groups linked to the transition metal metal M being n.

In the present invention, the term "substituted" in the expression "substituted or unsubstituted" refers to containing substituent(s), and the substituent(s) herein may be independently selected from halogen, hydroxy, C1-C6 alkyl, halo-substituted C1-C6 alkyl, C1-C6 alkoxy and halo-substituted C1-C6 alkoxy. Preferably, the halo is selected from fluoro, chloro, bromo, or iodo.

In the present invention, alkyl (such as C1-C6 alkyl or C1-C10 alkyl) may be independently selected from methyl, ethyl, n-propyl, isopropyl, n-butyl, isobutyl, n-pentyl, isopentyl, n-hexyl, isohexyl and 3,3-dimethyl butyl.

In the present invention, alkoxy (such as C1-C6 alkoxy) may be independently selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy.

In the present invention, aryl (such as C6-C8 aryl, C6-C15 aryl or C6-C20 aryl) may be independently selected from phenyl, 4-methyl phenyl, 4-ethyl phenyl, dimethyl phenyl, vinyl phenyl, anthryl, naphthyl, or biphenyl and the like.

In the present invention, the halogen is independently selected from fluorine, chlorine, bromine and iodine.

In a further preferred embodiment, the phosphine-phenol transition metal complex is selected from a group consisting of the following complexes,
complex 1: complex of formula (II), wherein M is Ti, R₁₁-R₁₅ are H, X is Cl, n=1;
complex 2: complex of formula (II), wherein M is Ti, R₁₁-R₁₅ are H, X is Cl, n=2;
complex 3: complex of formula (II), wherein M is Ti, R₁₁-R₁₅ are H, X is methyl, n=1;
complex 4: complex of formula (II), wherein M is Ti, R₁₁-R₁₅ are H, X is -CH₂C₆H₅, n=2;
complex 5: complex of formula (II), wherein M is Ti, R₁₁, R₁₂, R₁₄ and R₁₅ are H, R₁₃ is -CF₃, X is Cl, n=1;
complex 6: complex of formula (II), wherein M is Ti, R₁₁, R₁₂, R₁₄ and R₁₅ are H, R₁₃ is -CF₃, X is Cl, n=2;
complex 7: complex of formula (II), wherein M is Ti, R₁₁, R₁₂, R₁₄ and R₁₅ are H, R₁₃ is -CF₃, X is methyl, n=2;
complex 8: complex of formula (II), wherein M is Ti, R₁₁, R₁₂, R₁₄ and R₁₅ are H, R₁₃ is -CF₃, X is -CH₂C₆H₅, n=2;
complex 9: complex of formula (II), wherein M is Ti, R₁₂-R₁₅ are H, R₁₁ is methoxy, X is Cl, n=1;
complex 10: complex of formula (II), wherein M is Ti, R₁₂-R₁₅ are H, R₁₁ is methoxy, X is Cl, n=2;
complex 11: complex of formula (II), wherein M is Ti, R₁₂-R₁₅ are H, R₁₁ is methoxy, X is methyl, n=2;
complex 12: complex of formula (II), wherein M is Ti, R₁₂-R₁₅ are H, R₁₁ is methoxy, X is - CH₂C₆H₅, n=2;
complex 13: complex of formula (II), wherein M is Ti, R₁₁, R₁₂, R₁₄ and R₁₅ are H, R₁₃ is methoxy, X is Cl, n=1;
complex 14: complex of formula (II), wherein M is Ti, R₁₁, R₁₂, R₁₄ and R₁₅ are H, R₁₃ is methoxy, X is Cl, n=2;
complex 15: complex of formula (II), wherein M is Ti, R₁₁, R₁₂, R₁₄ and R₁₅ are H, R₁₃ is methoxy, X is methyl, n=2;
complex 16: complex of formula (II), wherein M is Ti, R₁₁, R₁₂, R₁₄ and R₁₅ are H, R₁₃ is methoxy, X is -CH₂C₆H₅, n=2;
complex 17: complex of formula (II), wherein M is Ti, R₁₁, R₁₃ and R₁₅ are H, R₁₂ and R₁₄ are - CF₃, X is Cl, n=1;
complex 18: complex of formula (II), wherein M is Ti, R₁₁, R₁₃ and R₁₅ are H, R₁₂ and R₁₄ are - CF₃, X is Cl, n=2;
complex 19: complex of formula (II), wherein M is Ti, R₁₁, R₁₃ and R₁₅ are H, R₁₂ and R₁₄ are - CF₃, X is methyl, n=2;
complex 20: complex of formula (II), wherein M is Ti, R₁₁, R₁₃ and R₁₅ are H, R₁₂ and R₁₄ are - CF₃, X is -CH₂C₆H₅, n=2;
complex 21: complex of formula (II), wherein M is Ti, R₁₁, R₁₃ and R₁₅ are H, R₁₂ and R₁₄ are methyl, X is Cl, n=1;
complex 22: complex of formula (II), wherein M is Ti, R₁₁, R₁₃ and R₁₅ are H, R₁₂ and R₁₄ are methyl, X is Cl, n=2;
complex 23: complex of formula (II), wherein M is Ti, R₁₁, R₁₃ and R₁₅ are H, R₁₂ and R₁₄ are methyl, X is methyl, n=2;
complex 24: complex of formula (II), wherein M is Ti, R₁₁, R₁₃ and R₁₅ are H, R₁₂ and R₁₄ are methyl, X is -CH₂C₆H₅, n=2;
complex 25: complex of formula (II), wherein M is Zr, R₁₁-R₁₅ are H, X is Cl, n=2;
complex 26: complex of formula (II), wherein M is Zr, R₁₁-R₁₅ are H, X is methyl, n=2;
complex 27: complex of formula (II), wherein M is Zr, R₁₁-R₁₅ are H, X is -CH₂C₆H₅, n=2;
complex 28: complex of formula (II), wherein M is Zr, R₁₁, R₁₂, R₁₄ and R₁₅ are H, R₁₃ is -CF₃, X is Cl, n=2;
complex 29: complex of formula (II), wherein M is Zr, R₁₁, R₁₂, R₁₄ and R₁₅ are H, R₁₃ is -CF₃, X is methyl, n=2;
complex 30: complex of formula (II), wherein M is Zr, R₁₁, R₁₂, R₁₄ and R₁₅ are H, R₁₃ is -CF₃, X is -CH₂C₆H₅, n=2;
complex 31: complex of formula (II), wherein M is Zr, R₁₂-R₁₅ are H, R₁₁ is methoxy, X is Cl, n=2;
complex 32: complex of formula (II), wherein M is Zr, R₁₂-R₁₅ are H, R₁₁ is methoxy, X is methyl, n=2;
complex 33: complex of formula (II), wherein M is Zr, R₁₂-R₁₅ are H, R₁₁ is methoxy, X is - CH₂C₆H₅, n=2;
complex 34: complex of formula (II), wherein M is Zr, R₁₁, R₁₂, R₁₄ and R₁₅ are H, R₁₃ is methoxy, X is Cl, n=2;
complex 35: complex of formula (II), wherein M is Zr, R₁₁, R₁₂, R₁₄ and R₁₅ are H, R₁₃ is methoxy, X is methyl, n=2;
complex 36: complex of formula (II), wherein M is Zr, R₁₁, R₁₂, R₁₄ and R₁₅ are H, R₁₃ is methoxy, X is -CH₂C₆H₅, n=2;
complex 37: complex of formula (II), wherein M is Zr, R₁₁, R₁₃ and R₁₅ are H, R₁₂ and R₁₄ are - CF₃, X is Cl, n=2;
complex 38: complex of formula (II), wherein M is Zr, R₁₁, R₁₃ and R₁₅ are H, R₁₂ and R₁₄ are - CF₃, X is methyl, n=2;
complex 39: complex of formula (II), wherein M is Zr, R₁₁, R₁₃ and R₁₅ are H, R₁₂ and R₁₄ are - CF₃, X is -CH₂C₆H₅, n=2;
complex 40: complex of formula (II), wherein M is Zr, R₁₁, R₁₃ and R₁₅ are H, R₁₂ and R₁₄ are methyl, X is Cl, n=2;
complex 41: complex of formula (II), wherein M is Zr, R₁₁, R₁₃ and R₁₅ are H, R₁₂ and R₁₄ are methyl, X is methyl, n=2;
complex 42: complex of formula (II), wherein M is Zr, R₁₁, R₁₃ and R₁₅ are H, R₁₂ and R₁₄ are methyl, X is -CH₂C₆H₅, n=2;
complex 43: complex of formula (II), wherein M is Hf, R₁₁-R₁₅ are H, X is Cl, n=2;
complex 44: complex of formula (II), wherein M is Hf, R₁₁-R₁₅ are H, X is methyl, n=2;
complex 45: complex of formula (II), wherein M is Hf, R₁₁-R₁₅ are H, X is -CH₂C₆H₅, n=2;
complex 46: complex of formula (II), wherein M is Hf, R₁₁, R₁₂, R₁₄ and R₁₅ are H, R₁₃ is -CF₃, X is Cl, n=2;
complex 47: complex of formula (II), wherein M is Hf, R₁₁, R₁₂, R₁₄ and R₁₅ are H, R₁₃ is methoxy, X is Cl, n=2;
complex 48: complex of formula (II), wherein M is Hf, R₁₁, R₁₃ and R₁₅ are H, R₁₂ and R₁₄ are - CF₃, X is Cl, n=2;
complex 49: complex of formula (II), wherein M is Hf, R₁₁, R₁₃ and R₁₅ are H, R₁₂ and R₁₄ are methyl, X is Cl, n=2;
complex 50: complex of formula (II), wherein M is Hf, R₁₁, R₁₃ and R₁₅ are H, R₁₂ and R₁₄ are - CF₃, X is -CH₂C₆H₅, n=2;
complex 51: complex of formula (II), wherein M is Hf, R₁₁, R₁₂, R₁₄ and R₁₅ are H, R₁₃ is -CF₃, X is -CH₂C₆H₅, n=2;
complex 52: complex of formula (II), wherein M is Hf, R₁₁, R₁₂, R₁₄ and R₁₅ are H, R₁₃ is methoxy, X is -CH₂C₆H₅, n=2;
complex 53: complex of formula (II), wherein M is Hf, R₁₁, R₁₃ and R₁₅ are H, R₁₂ and R₁₄ are methyl, X is -CH₂C₆H₅, n=2;
complex 54: complex of formula (II), wherein M is Hf, R₁₁, R₁₃ and R₁₅ are H, R₁₂ and R₁₄ are - CF₃, X is -CH₂C₆H₅, n=2.

A method for preparing the above-mentioned phosphine-phenol transition metal complex may comprise:
(1) reacting a compound of formula (III) with a compound of formula (IV), to obtain a ligand of formula (V);
(2) reacting the ligand with a hydrogen abstracting agent, and then reacting with an M metal compound, the transition metal M in the M metal compound being selected from Group IVB metals; 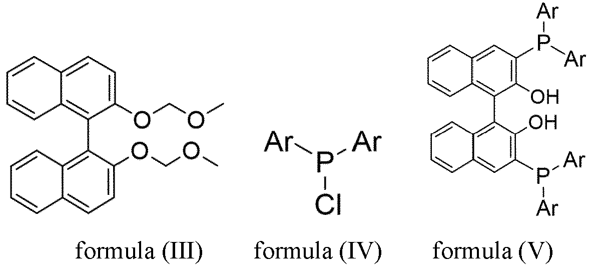 wherein, the definition of Ar is the same as described above.

In a more preferred embodiment, the reaction process of step (1) is shown in the following equation. 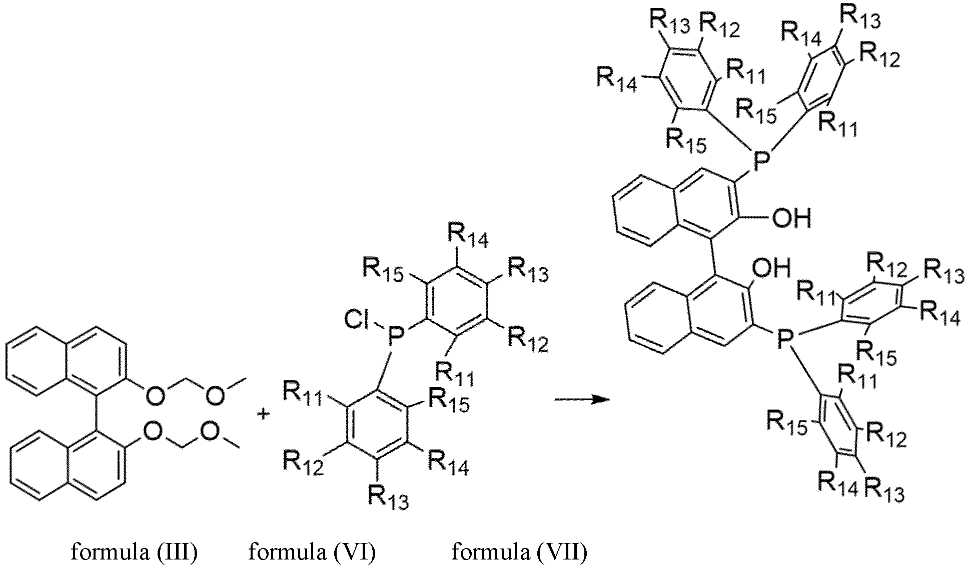 wherein, the definitions of R₁₁-R₁₅ are the same as described above.

In the method of the present invention, in a preferred case, the transition metal M in the M metal compound is selected from titanium, zirconium and hafnium. Further preferably, the M metal compound is selected from at least one of titanium tetrachloride, bis(tetrahydrofuran) titanium tetrachloride, tri(tetrahydrofuran) titanium trichloride, zirconium tetrachloride, bis(tetrahydrofuran) zirconium tetrachloride, hafnium tetrachloride, and bis(tetrahydrofuran) hafnium tetrachloride.

In the method of the present invention, in a preferred case, the reaction process of step (1) comprises preferably first reacting a compound of formula (III) with a hydrogen abstracting agent, and then reacting with a compound of formula (IV).

In step (1) and step (2), the hydrogen abstracting agents used may be the same or different, and are each independently selected from at least one of NaH, KH, n-butyl lithium and methyl lithium.

In a specific embodiment, the preparation process of step (1) comprises: under a protective gas (such as nitrogen) atmosphere, dissolving a compound of formula (III) in anhydrous diethyl ether, adding a hydrogen abstracting agent (such as n-butyl lithium) at room temperature, stirring at room temperature, adding tetrahydrofuran, and further stirring the obtained black solution containing precipitate; next, adding a compound of formula (VI), stirring at room temperature, and quenching with the addition of NH₄Cl aqueous solution; then extracting the organic phase with ethyl acetate, drying the obtained organic phase over anhydrous sodium sulfate, and recrystallizing with dichloromethane/hexane to obtain a yellow crystalline compound; afterwards, adding methanol and concentrated hydrochloric acid, reacting under reflux, after the completion of the reaction, removing the organic solvent, dissolving the product in ethyl acetate, neutralizing with NaHCO₃ aqueous solution, and extracting the organic phase, followed by drying, filtration, concentration, and column chromatography to obtain a ligand of formula (VII).

In a specific embodiment, the preparation process of step (2) comprises: under a protective gas (such as nitrogen) atmosphere, dissolving the ligand obtained in step (1) in tetrahydrofuran, adding excess hydrogen abstracting agent (such as NaH, KH and the like), stirring at room temperature, filtering to remove the hydrogen abstracting agent, then adding a tetrahydrofuran solution of M metal compound, reacting overnight, removing the solvent by suction, dissolving in dichloromethane, filtering to remove the filter cake, concentrating the filtrate, and recrystallizing with heptane, to obtain the phosphine-phenol transition metal complex of the present invention.

The present invention also provides use of the above-mentioned phosphine-phenol transition metal complex in olefin polymerization. Preferably, the olefins include ethylene, α-olefins, and cycloolefins. The cycloolefins include but are not limited to: cyclobutene, cyclopentene, cyclohexene, cycloheptene, cyclooctene, norbornene and the like.

The present invention also provides an olefin polymerization catalyst, comprising the above-mentioned phosphine-phenol transition metal complex as a primary catalyst.

The olefin polymerization catalyst further comprises a cocatalyst. The cocatalyst is organoaluminum compound and/or organoboron compound.

The organoaluminum compound is selected from alkylaluminoxane or organoaluminum compound of general formula AlRₙX¹₃₋ₙ (alkyl aluminum or alkyl aluminum halide). In the general formula AlRₙX¹₃₋ₙ, R is H, C1-C20 hydrocarbyl (such as C1-C10 hydrocarbyl) or C1-C20 hydrocarbyloxy (such as C1-C10 hydrocarbyloxy), preferably C1-C20 alkyl (such as C1-C10 alkyl), C1-C20 alkoxy (such as C1-C10 alkoxy), C7-C20 aralkyl (such as C7-C15 aralkyl) or C6-C20 aryl (such as C6-C15 aryl); X¹ is halogen, preferably chlorine or bromine; 0<n≤3. Specific examples of the organoaluminum compound include but are not limited to: trimethyl aluminum, triethyl aluminum, triisobutyl aluminum, tri-n-hexyl aluminum, trioctyl aluminum, diethylaluminum hydride, diisobutyl aluminum hydride, diethylaluminum chloride, diisobutyl aluminum chloride, ethylaluminum sesquichloride, ethyl aluminum dichloride, methyl aluminoxane (MAO) and modified methyl aluminoxane (MMAO). Preferably, the organoaluminum compound is methyl aluminoxane (MAO).

The organoboron compound is selected from aryl boron and/or borate. The aryl boron is preferably substituted or unsubstituted phenyl boron, more preferably tri(pentafluorophenyl) boron. The borate is preferably N,N-dimethylanilinium tetrakis(pentafluorophenyl) borate and/or triphenylmethyl tetrakis(pentafluorophenyl) borate.

According to the preferred embodiment of the present invention, when the cocatalyst is organoaluminum compound, the molar ratio of the aluminum in the cocatalyst to the transition metal M in the primary catalyst is (10-10⁷):1, for example, 10:1, 20:1, 50:1, 100:1, 200:1, 300:1, 500:1, 700:1, 800:1, 1000:1, 2000:1, 3000:1, 5000:1, 10000:1, 100000:1, 1000000:1, 10000000:1 and any value therebetween, preferably (10-100000):1, more preferably (100-10000):1; when the cocatalyst is organoboron compound, the molar ratio of the boron in the cocatalyst to the transition metal M in the primary catalyst is (0.1-1000):1, for example, 0.1:1, 0.2:1, 0.5:1, 1:1, 2:1, 3:1, 5:1, 8:1, 10:1, 20:1, 50:1, 100:1, 200:1, 300:1, 500:1, 700:1, 800:1, 1000:1 and any value therebetween, preferably (0.1-500):1.

In the present invention, the olefins are C2-C16 olefins. Preferably, the olefins are ethylene or α-olefins having 3 to 16 carbon atoms.

The present invention also provides an olefin polymerization method, comprising carrying out olefin polymerization reaction in the presence of the above-mentioned olefin polymerization catalyst. The olefin polymerization reaction may be homopolymerization or copolymerization.

The olefin polymerization reaction temperature may be -78°C~200°C, preferably -20°C ~150°C, the pressure may be 0.01~10MPa, preferably 0.01~5MPa. Here, the term "pressure" refers to ethylene pressure in the polymerization system, expressed in absolute pressure.

In the olefin polymerization method according to the present invention, the olefins are C2-C16 olefins.

According to an embodiment of the present invention, the olefins include ethylene.

According to an embodiment of the present invention, the olefins include ethylene, propylene, α-olefin and cycloolefins.

According to an embodiment of the present invention, the olefin polymerization reaction is carried out by olefin monomers in a solvent, and the solvent for polymerization is selected from one or more of alkanes, aromatic hydrocarbons and halogenated hydrocarbons. Specifically, the solvent for polymerization is selected from one or more of hexane, pentane, heptane, benzene, toluene, dichloromethane, chloroform, chlorobenzene and dichloroethane, preferably one or more of hexane, toluene and heptane.

In the present invention, alkyl refers to linear alkyl, branched alkyl, or cyclic alkyl, including but not limited to: methyl, ethyl, n-propyl, isopropyl, n-butyl, sec-butyl, isobutyl, tert-butyl, n-pentyl, isopentyl, tert-pentyl, neopentyl, n-hexyl, n-heptyl, n-octyl, n-decyl, cyclopropyl, cyclopentyl, cyclohexyl, 4-methylcyclohexyl, 4-ethylcyclohexyl, 4-n-propylcyclohexyl, and 4-n-butylcyclohexyl.

In the present invention, examples of aryl include, but are not limited to: phenyl, 4-methylphenyl, 4-ethylphenyl, dimethylphenyl, vinylphenyl.

In the present invention, alkenyl refers to linear alkenyl, branched alkenyl or cycloalkenyl, including but not limited to: vinyl, allyl, butenyl.

In the present invention, examples of aralkyl include, but are not limited to: phenylmethyl, phenylethyl, phenyl-n-propyl, phenyl-isopropyl, phenyl-n-butyl and phenyl-tert-butyl.

In the present invention, examples of alkaryl include, but are not limited to: tolyl, ethylphenyl, n-propylphenyl, isopropylphenyl, n-butylphenyl and tert-butylphenyl.

The phosphine-phenol transition metal complex and preparation method therefor and use thereof according to the present invention are further illustrated by the following examples. The examples, which are implemented on the premise of the technical solution of the present invention, give detailed embodiments and specific operation processes. However, the protection scope of the present invention is not limited to the following examples.

The experimental methods in the following examples, unless otherwise specified, are conventional methods in the art. The experimental materials used in the following examples are commercially available unless otherwise specified.

The analytical characterization equipment and test methods used in the following Examples and Comparative Examples are as follows:
(1) Nuclear magnetic resonance instrument: Bruker DMX 300 (300 MHz), with tetramethylsilane (TMS) as the internal standard.
(2) Molecular weight and molecular weight distribution PDI (PDI = Mw/Mn) of the polymer: the measurement is carried out at 150 °C using a PL-GPC220 chromatograph with trichlorobenzene as the solvent (in which standard sample: PS, flow rate: 1.0 mL/min, chromatographic column: 3×PLgel 10um M1×ED-B, 300 ×7.5nm).
(3) Activity measurement method: the polymer is washed with hydrochloric acid-ethanol solution, dried in vacuum, and weighed. The polymerization activity is calculated as: polymer weight (g)/metal (mol) ×60/polymerization time (min).
(4) Structure analysis of the complex: single crystal testing analysis, using Rigaku RAXIS Rapid IP diffractometer.
(5) Analysis of the comonomer content of the polymer: the polymer sample is dissolved in 1,2,4-trichlorobenzene at 120°C, and analyzed by ¹H NMR, ¹³C NMR spectrum on a 400MHz Bruker Avance 400 nuclear magnetic resonance spectrometer using a 10mm PASEX 13 probe.

### Example 1

### complex 2: complex of formula (II), wherein M is Ti, R₁₁-R₁₅ are H, X is Cl, n=2.

Under a nitrogen atmosphere, a compound of formula (III) (11.23g, 30mmol, S configuration) was dissolved in anhydrous diethyl ether (150mL), an n-butyl lithium solution (2.7M, 33.3mL, 90mmol) was added dropwise at room temperature, and the solution was stirred at room temperature for 4h. Tetrahydrofuran (150mL) was added, and the obtained black solution containing precipitate was further stirred for 1h. Diphenylphosphine chloride (PPh₂Cl) (90mmol, 16.7mL) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a yellow crystalline compound (15.54 g). Methanol (100mL), 5mL of concentrated hydrochloric acid (37wt%) were added, and reacted under reflux for 16h. After the reaction as tracked using thin-layer chromatography (TLC) was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₁, with a yield of 67 %. ¹H NMR (400 MHz, CDCl₃): δ = 5.41 (s, 2H), 7.13-7.15 (m, 2H), 7.24-7.29 (m, 4H), 7.38-7.45 (m, 22H), 7.62-7.64 (m, 2H). ³¹P NMR (162 MHz, CDCl₃): δ = -17.19 (s). High resolution mass spectrometry test: theoretical calculation value: 654.19; test value: 655.20.

Under a nitrogen atmosphere, the ligand L₁ (0.654g, 1mmol) was dissolved in tetrahydrofuran, excess NaH (0.072g, 3mmol) was added, the solution was stirred at room temperature for 10 h, and then NaH was removed by filtration. A solution of TiCl₄(THF)₂ (0.334g, 1mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a red complex 2 (its single crystal structural diagram is shown in Figure 1), with a yield of 74%. ¹HNMR (300 MHz, CDCl₃): δ = 6.80-6.91 (m,4H), 6.93-7.15 (m, 22H), 7.18-7.20 (m, 4H), 7.26-7.36 (m, 22H), 7.47-7.60 (m, 4H), 7.88-7.92 (m, 4H). Elemental analysis test C₈₈H₆₀Cl₄O₄P₄Ti₂: theoretical calculation value: C, 68.51; H, 3.92; test value: C, 68.37; H, 4.14.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 7.7mg (5µmol) of complex 2 were added. The reaction was carried out with stirring vigorously at 30°C for 20min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 2

### complex 2: complex of formula (II), wherein M is Ti, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 7.7mg (5µmol) of complex 2 were added. The reaction was carried out with stirring vigorously at 30°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 3

### complex 2: complex of formula (II), wherein M is Ti, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 7.7mg (5µmol) of complex 2 were added. The reaction was carried out with stirring vigorously at 80°C for 30min while maintaining an ethylene pressure of 10 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 4

### complex 2: complex of formula (II), wherein M is Ti, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 10 mL of 1-octene, 7.7mg (5µmol) of complex 2 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Example 5

### complex 2: complex of formula (II), wherein M is Ti, R₁₁-R₁₅ are H, X is Cl, n=2.

A 100mL glass polymerization kettle equipped with mechanical stirrer was dried continuously at 130°C for 6h, vacuumized while hot and purged 3 times with N₂. To the polymerization kettle, 50mL of toluene was charged, and methylaluminoxane (6.5 mL, 10 mmol), 0.5g of norbornene, 3.1mg (2µmol) of complex 2 were added. The reaction was carried out with stirring vigorously at 30°C for 10min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Example 6

### complex 2: complex of formula (II), wherein M is Ti, R₁₁-R₁₅ are H, X is Cl, n=2.

A 100mL glass polymerization kettle equipped with mechanical stirrer was dried continuously at 130°C for 6h, vacuumized while hot and purged 3 times with N₂. To the polymerization kettle, 50mL of toluene was charged, and methylaluminoxane (6.5 mL, 10 mmol), 0.5g of norbornene, 3.1mg (2µmol) of complex 2 were added. The reaction was carried out with stirring vigorously at 50°C for 10min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Example 7

### complex 2: complex of formula (II), wherein M is Ti, R₁₁-R₁₅ are H, X is Cl, n=2.

A 100mL glass polymerization kettle equipped with mechanical stirrer was dried continuously at 130°C for 6h, vacuumized while hot and purged 3 times with N₂. To the polymerization kettle, 50mL of toluene was charged, and methylaluminoxane (6.5 mL, 10 mmol), 0.5g of norbornene, 3.1mg (2µmol) of complex 2 were added. The reaction was carried out with stirring vigorously at 70°C for 10min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Example 8

### complex 2: complex of formula (II), wherein M is Ti, R₁₁-R₁₅ are H, X is Cl, n=2.

A 100mL glass polymerization kettle equipped with mechanical stirrer was dried continuously at 130°C for 6h, vacuumized while hot and purged 3 times with N₂. To the polymerization kettle, 50mL of toluene was charged, and methylaluminoxane (6.5 mL, 10 mmol), 5.0g of norbornene, 3.1mg (2µmol) of complex 2 were added. The reaction was carried out with stirring vigorously at 75°C for 10min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution, polymerization activity and comonomer content of the obtained polymer are shown in Table 1.

### Example 9

### complex 25: complex of formula (II), wherein M is Zr, R₁₁-R₁₅ are H, X is Cl, n=2.

A ligand L₁ was prepared according to the method of Example 1.

Under a nitrogen atmosphere, the ligand L₁ (0.654g, 1mmol) was dissolved in tetrahydrofuran, excess NaH (0.072g, 3mmol) was added, the solution was stirred at room temperature for 10 h, and NaH was removed by filtration. A solution of (THF)ZrCl₂ (0.377g, 1mmol) in tetrahydrofuran (-78°C) was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a white complex 25, with a yield of 72%. ¹HNMR (300 MHz, CDCl₃): δ: 6.81-6.92 (m,4H), 7.04-7.10 (m, 4H), 7.13-7.20 (m,4H), 7.27-7.29 (m, 16H), 7.38-7.45 (m, 20 H), 7.57-7.72 (m, 8 H), 7.78-7.87 (m, 2H), 7.95-8.00 (m, 2H). ³¹P NMR (162 MHz, CDCl₃): δ = -1.96 (s). Elemental analysis test C₈₈H₆₀Cl₄O₄P₄Zr₂: theoretical calculation value: C, 64.86; H, 3.71; test value: C, 64.69; H, 3.93.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 8.1mg (5µmol) of complex 25 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 10

### complex 25: complex of formula (II), wherein M is Zr, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 8.1mg (5µmol) of complex 25 were added. The reaction was carried out with stirring vigorously at 30°C for 30min while maintaining a propylene pressure of 2.0 atm. The reaction solution was neutralized with a 10 wt% hydrochloric acid acidified ethanol solution to obtain polypropylene. According to the analysis of nuclear magnetic carbon spectrum, the obtained polypropylene had a syndiotacticity of 41.2 %. The polymer was dried and weighed for the test of polymerization activity. The test data of weight average molecular weight, molecular weight distribution and polymerization activity of the obtained polymer are shown in Table 1.

### Example 11

### complex 43: complex of formula (II), wherein M is Hf, R₁₁-R₁₅ are H, X is Cl, n=2.

A ligand L₁ was prepared according to the method of Example 1.

Under a nitrogen atmosphere, the ligand L₁ (0.654g, 1mmol) was dissolved in tetrahydrofuran, excess NaH (0.072g, 3mmol) was added, the solution was stirred at room temperature for 10 h, and then NaH was removed by filtration. A solution of (THF)₂HfCl₄ (0.464 g, 1mmol) in tetrahydrofuran (-78°C) was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a white complex 43, with a yield of 78%. Elemental analysis test C₈₈H₆₀Cl₄Hf₂O₄P₄: theoretical calculation value: C, 58.59; H, 3.35; test value: C, 58.71; H, 3.52.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 9.0mg (5µmol) of complex 43 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 12

### complex 43: complex of formula (II), wherein M is Hf, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 9.0mg (5µmol) of complex 43 were added. The reaction was carried out with stirring vigorously at 50°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 13

### complex 43: complex of formula (II), wherein M is Hf, R₁₁-R₁₅ are H, X is Cl, n=2.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 9.0mg (5µmol) of complex 43 were added. The reaction was carried out with stirring vigorously at 30°C for 30min while maintaining a propylene pressure of 2.0 atm. The reaction solution was neutralized with a 10 wt% hydrochloric acid acidified ethanol solution to obtain polypropylene. The polymer was dried and weighed for the test of polymerization activity. The test data of weight average molecular weight, molecular weight distribution and polymerization activity of the obtained polymer are shown in Table 1.

### Example 14

### complex 18: complex of formula (II), wherein M is Ti, R₁₁, R₁₃ and R₁₅ are H, R₁₂ and R₁₄ are -CF₃, X is Cl, n=2.

Its synthesis steps were similar to those of complex 2 in Example 1, except for replacing diphenylphosphine chloride with bis(3,5-di(trifluoromethyl)phenyl)phosphine chloride.

Under a nitrogen atmosphere, a compound of formula (III) (3.74g, 10mmol, R configuration) was dissolved in anhydrous diethyl ether (120mL), an n-butyl lithium solution (2.7M, 11.1mL, 30mmol) was added dropwise at room temperature, and the solution was stirred at room temperature for 4h. Tetrahydrofuran (120mL) was added, and the obtained black solution containing precipitate was further stirred for 1h. Bis(3,5-di(trifluoromethyl)phenyl)phosphine chloride (30mmol, 14.78g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₂, 4.78g, with a yield of 40%. ¹H NMR (400 MHz, CDCl₃) δ 5.14(s, 2H), 7.08-7.11 (m, 2H), 7.41-7.46 (m, 4H), 7.63 (d, 2H), 7.78-7.81(m, 2H), 7.84(d, 4H), 7.86(d, 4H), 7.91(s, 2H), 7.94(s, 2H). ¹H NMR (400 MHz, DMSO) δ 9.10 (s, 2H), 8.17 (s, 2H), 8.13 (s, 2H), 8.08 (dd, J = 13.3, 6.5 Hz, 8H), 7.81 (d, J = 7.6 Hz, 2H), 7.56 (d, J = 8.4 Hz, 2H), 7.27 (d, 4H), 6.90 (d, J = 8.2 Hz, 2H). High resolution mass spectrometry test: theoretical calculation value: 1198.09; test value: 1198.95. ³¹P NMR (162 MHz, DMSO): δ = -8.37 (s).

Under a nitrogen atmosphere, the ligand L₂ (1.20g, 1mmol) was dissolved in tetrahydrofuran, excess NaH (0.072g, 3mmol) was added, the solution was stirred at room temperature for 10 h, and then NaH was removed by filtration. A solution of TiCl₄(THF)₂ (0.334g, 1mmol) in tetrahydrofuran was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a red complex 18, with a yield of 72%. ¹H NMR (400 MHz, CDCl₃) δ 6.78-7.04 (m, 4H), 7.08-7.10 (d, 4H), 7.14-7.18 (m, 2H), 7.24-7.31 (m, 6H), 7.41-7.48(m, 6H), 7.64(d, 4H), 7.78-7.81(m, 4H), 7.83(d, 4H), 7.86(d, 4H), 7.92(d, 4H), 7.99-8.11(m, 2H). ³¹P NMR (162 MHz, DMSO): δ = -9.22 (s). Elemental analysis test C₁₀₄H₄₄Cl₄F₄₈O₄P₄Ti₂: theoretical calculation value: C, 47.48; H, 1.69; test value: C, 47.57; H, 1.83.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (3.3 mL, 5 mmol), 6.6mg (2.5µmol) of complex 18 were added. The reaction was carried out with stirring vigorously at 30°C for 20min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 15

### complex 37: complex of formula (II), wherein M is Zr, R₁₁, R₁₃ and R₁₅ are H, R₁₂ and R₁₄ are -CF₃, X is Cl, n=2.

A ligand L₂ was prepared according to the method of Example 1.

Under a nitrogen atmosphere, the ligand L₂ (1.20g, 1mmol) was dissolved in tetrahydrofuran, excess NaH (0.072g, 3mmol) was added, the solution was stirred at room temperature for 10 h, and then NaH was removed by filtration. A solution of (THF)ZrCl₂ (0.377g, 1mmol) in tetrahydrofuran (-78 °C) was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a white complex 37, with a yield of 74%. Elemental analysis test C₁₀₄H₄₄Cl₄F₄₈O₄P₄Zr₂: theoretical calculation value: C, 45.97; H, 1.63; test value: C,45.76; H, 1.92.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (3.3 mL, 5 mmol), 6.8mg (2.5µmol) of complex 37 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 16

### complex 40: complex of formula (II), wherein M is Zr, R₁₁, R₁₃ and R₁₅ are H, R₁₂ and R₁₄ are methyl, X is Cl, n=2.

Under a nitrogen atmosphere, a compound of formula (III) (3.74g, 10mmol, R configuration) was dissolved in anhydrous diethyl ether (120mL), an n-butyl lithium solution (2.5M, 12mL, 30mmol) was added dropwise at room temperature, and the solution was stirred at room temperature for 4h. Tetrahydrofuran (120mL) was added, and the obtained black solution containing precipitate was further stirred for 1h. Di(3,5-dimethylphenyl)phosphine chloride (30mmol, 8.30g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₃, with a yield of 58%. ¹H NMR (400 MHz, CDCl₃) δ: 2.28 (s, 24H), 5.43 (s, 2H), 6.96-7.05 (m, 12H), 7.17 (m, 2H), 7.24-7.29 (m, 4H), 7.43 (d, 2H), 7.65 (m, 2H). High resolution mass spectrometry test: theoretical calculation value: 766.31; test value: 767.32. ³¹P NMR (162 MHz, CDCl₃) δ = -15.4 (s).

Under a nitrogen atmosphere, the ligand L₃ (0.77g, 1mmol) was dissolved in tetrahydrofuran, excess NaH (0.072g, 3mmol) was added, the solution was stirred at room temperature for 10 h, and then NaH was removed by filtration. A solution of (THF)ZrCl₂ (0.377g, 1mmol) in tetrahydrofuran (-78°C) was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a white complex 40, with a yield of 72%. Elemental analysis test C₁₀₄H₉₂Cl₄O₄P₄Z₂: theoretical calculation value: C, 67.37; H, 5.00; test value: C,67.46; H, 5.32.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (3.3 mL, 5 mmol), 4.6mg (2.5µmol) of complex 40 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Example 17

### complex 34: complex of formula (II), wherein M is Zr, R₁₁, R₁₂, R₁₄ and R₁₅ are H, R₁₃ is methoxy, X is Cl, n=2.

Under a nitrogen atmosphere, a compound of formula (III) (3.74g, 10mmol, S configuration) was dissolved in anhydrous diethyl ether (120mL), an n-butyl lithium solution (2.7M, 11.1mL, 30mmol) was added dropwise at room temperature, and the solution was stirred at room temperature for 4h. Tetrahydrofuran (120mL) was added, and the obtained black solution containing precipitate was further stirred for 1h. Di(4-methoxy)phosphine chloride (30mmol, 8.42g) was added at 0°C. The solution was stirred at room temperature for 1h, and then quenched with the addition of NH₄Cl aqueous solution (5mL). The organic phase was extracted with ethyl acetate, and the obtained organic phase was dried over anhydrous sodium sulfate, recrystallized with dichloromethane/hexane, to obtain a white crystalline compound. Methanol (100mL), 5mL of concentrated hydrochloric acid (37wt%) were added, and reacted under reflux for 16h. After the reaction as tracked using TLC was completed, the organic solvent was removed, and the product was dissolved in ethyl acetate, and neutralized with NaHCO₃ aqueous solution. The organic phase was extracted, dried over anhydrous MgSO₄, filtered, concentrated, and separated by column chromatography (dichloromethane as solvent) to obtain ligand L₄, with a yield of 56%.

Under a nitrogen atmosphere, the ligand L₄ (0.77g, 1mmol) was dissolved in tetrahydrofuran, excess NaH (0.072g, 3mmol) was added, the solution was stirred at room temperature for 10 h, and then NaH was removed by filtration. A solution of (THF)ZrCl₂ (0.377g, 1mmol) in tetrahydrofuran (-78°C) was added dropwise, and reacted overnight at room temperature. The solvent was removed by suction, and the product was dissolved in dichloromethane (40mL). The solution was filtered to remove the filter cake, the filtrate was concentrated, and recrystallized with heptane to obtain a white complex 34, with a yield of 73%. Elemental analysis test C₉₆H₇₆Cl₄O₁₂P₄Zr₂: theoretical calculation value: C, 61.67; H, 4.10; test value: C,61.58; H, 4.33.

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (3.3 mL, 5 mmol), 4.7mg (2.5µmol) of complex 34 were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

### Comparative Example 1

A 1L stainless steel polymerization kettle equipped with mechanical stirrer was dried continuously at 130°C for 6h, vacuumized while hot and purged 3 times with N₂. To the polymerization kettle, 500mL of toluene was charged, and methylaluminoxane (6.5 mL, 10 mmol), 8.3mg (10µmol) of complex A (as to its synthesis process, please refer to the literature Organometallics, 2008, 27(2), 235-245) were added. The reaction was carried out with stirring vigorously at 30°C for 20min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

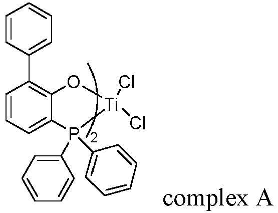

### Comparative Example 2

A 100mL glass polymerization kettle equipped with mechanical stirrer was dried continuously at 130°C for 6h, vacuumized while hot and purged 3 times with N₂. To the polymerization kettle, 50mL of toluene was charged, and methylaluminoxane (6.5 mL, 10 mmol), 0.5g of norbornene, 3.3mg (4.0µmol) of complex A were added. The reaction was carried out with stirring vigorously at 30°C for 10min while maintaining an ethylene pressure of 1.0 atm. The reaction solution was neutralized with a 10 wt% hydrochloric acid acidified ethanol solution to obtain a polymer. The polymer was dried and weighed for the test of polymerization activity. The test data of weight average molecular weight, molecular weight distribution and polymerization activity of the obtained polymer are shown in Table 1.

### Comparative Example 3

A 1L stainless steel polymerization kettle equipped with mechanical stirrer was dried continuously at 130°C for 6h, vacuumized while hot and purged 3 times with N₂. To the polymerization kettle, 500mL of hexane was charged, and methylaluminoxane (6.5 mL, 10 mmol), 8.3mg (10µmol) of complex B (as to its synthesis process, please refer to the literature Organometallics, 2008, 27(2), 235-245) were added. The reaction was carried out with stirring vigorously at 30°C for 30min while maintaining an ethylene pressure of 1.0 atm. The reaction solution was neutralized with a 10 wt% hydrochloric acid acidified ethanol solution to obtain polyethylene. The polymer was dried and weighed for the test of polymerization activity. The test data of weight average molecular weight, molecular weight distribution and polymerization activity of the obtained polymer are shown in Table 1.

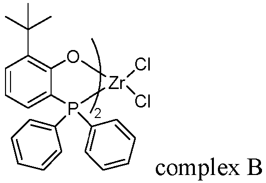

**Table 1**

| Example | Complex | Transition metal M in complex | Polymerization activity (g/molcat·h) | *M*_{w}×10⁻⁴ | *M*_{w}/*M*ₙ | Comonomer content (mol%) |
|---|---|---|---|---|---|---|
| Example 1 | complex 2 | Ti | 9.27×10⁵ | 12.2 | 2.0 | - |
| Example 2 | complex 2 | Ti | 2.62×10⁷ | 52.4 | 2.1 | - |
| Example 3 | complex 2 | Ti | 3.24×10⁶ | 11.4 | 2.4 | - |
| Example 4 | complex 2 | Ti | 1.42×10⁶ | 8.7 | 1.9 | 3.2 |
| Example 5 | complex 2 | Ti | 3.62×10⁵ | 4.2 | 1.2 | 12.2 |
| Example 6 | complex 2 | Ti | 4.14×10⁵ | 5.0 | 1.2 | 19.0 |
| Example 7 | complex 2 | Ti | 5.72×10⁵ | 6.3 | 1.2 | 29.1 |
| Example 8 | complex 2 | Ti | 5.13×10⁵ | 3.2 | 1.1 | 48.0 |
| Example 9 | complex 25 | Zr | 8.63×10⁷ | 8.6 | 2.5 | - |
| Example 10 | complex 25 | Zr | 6.74×10⁶ | 8.4 | 1.8 | - |
| Example 11 | complex 43 | Hf | 3.46×10⁶ | 63.2 | 4.0 | - |
| Example 12 | complex 43 | Hf | 5.25×10⁶ | 67.3 | 4.4 | - |
| Example 13 | complex 43 | Hf | 8.91×10⁵ | 4.8 | 1.9 | - |
| Example 14 | complex 18 | Ti | 1.13×10⁶ | 10.8 | 1.3 | - |
| Example 15 | complex 37 | Zr | 9.04×10⁷ | 9.2 | 2.3 | - |
| Example 16 | complex 40 | Zr | 7.42×10⁷ | 11.3 | 2.1 | - |
| Example 17 | complex 34 | Zr | 7.56×10⁷ | 14.6 | 2.0 | - |
| Comparative Example 1 | complex A | Ti | 2.24×10⁵ | 3.4 | 2.6 | - |
| Comparative Example 2 | complex A | Ti | 2.25×10⁵ | 2.7 | 1.6 | 10.1 |
| Comparative Example 3 | complex B | Zr | 9.82×10⁶ | 2.4 | 2.3 | - |

As can be seen from the data in Table 1, in the case where the complex contains the same metal and the polymerization conditions are comparable, when the metal complex of the present invention is used as a primary catalyst, a higher activity for olefin polymerization is achieved compared with the complex in the Comparative Examples, and the obtained polymer has an obviously higher molecular weight than that of the polymer obtained in the Comparative Examples.

The preferred embodiments of the present invention have been described above in detail, but the present invention is not limited thereto. Within the scope of the technical idea of the present invention, many simple modifications can be made to the technical solution of the present invention, including various technical features being combined in any other suitable way, and these simple modifications and combinations should also be regarded as the disclosure of the present invention, and all fall within the scope of the present invention.

## Claims

1. A phosphine-phenol transition metal complex, **characterized in that** it has a structural formula shown in formula (I), 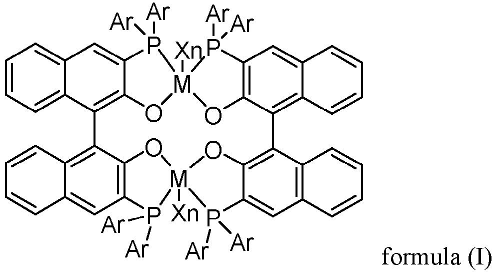
wherein, M is selected from Group IVB metals; Ar is selected from substituted or unsubstituted C6-C20 aryl; X is selected from halogen, C1-C10 hydrocarbyl, n is 1 or 2,
preferably, C6-C20 aryl is selected from phenyl, 4-methyl phenyl, 4-ethyl phenyl, dimethyl phenyl, vinyl phenyl, anthryl, naphthyl, or biphenyl;
preferably, for substituted C6-C20 aryl, the substituent thereof is selected from H, halogen, hydroxy, substituted or unsubstituted alkoxy, substituted or unsubstituted C1-C20 hydrocarbyl; preferably, alkoxy is C1-C6 alkoxy;
preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy;
preferably, the halo is selected from fluoro, chloro, bromo, or iodo;
preferably, C1-C10 hydrocarbyl is selected from C1-C8 hydrocarbyl; and/or
preferably, C1-C20 hydrocarbyl is selected from C1-C6 alkyl.

2. The phosphine-phenol transition metal complex as claimed in claim 1, **characterized in that** it has a structural formula shown in formula (II), 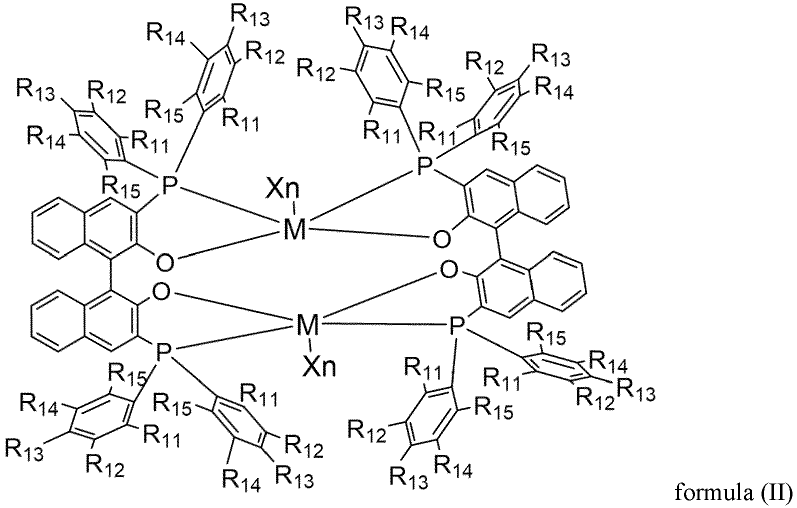 wherein, R₁₁-R₁₅ are each independently selected from H, halogen, hydroxy, substituted or unsubstituted alkoxy or substituted or unsubstituted C1-C20 hydrocarbyl; preferably, for substituted alkoxy and substituted C1-C20 hydrocarbyl, the substituents thereof are selected from halogen, hydroxy, C1-C6 alkyl, halo-substituted C1-C6 alkyl, C1-C6 alkoxy and halo-substituted C1-C6 alkoxy.

3. The phosphine-phenol transition metal complex as claimed in claim 1 or 2, **characterized in that**, M is selected from titanium, zirconium and hafnium.

4. The phosphine-phenol transition metal complex as claimed in claim 1 or 2, **characterized in that**, R₁₁-R₁₅ are each independently selected from H, halogen, hydroxy, substituted or unsubstituted alkoxy or substituted or unsubstituted C1-C10 alkyl and substituted or unsubstituted C6-C15 aryl; X is selected from halogen, C1-C8 hydrocarbyl;
preferably, for substituted alkoxy, substituted C1-C10 alkyl and substituted C6-C15 aryl, the substituents are independently selected from halogen, hydroxy, C1-C6 alkyl, halo-substituted C1-C6 alkyl, C1-C6 alkoxy and halo-substituted C1-C6 alkoxy;
preferably, the C1-C6 alkyl is selected from methyl, ethyl, n-propyl, isopropyl, n-butyl, isobutyl, n-pentyl, isopentyl, n-hexyl, isohexyl and 3,3-dimethyl butyl;
preferably, the C1-C6 alkoxy is selected from methoxy, ethoxy, n-propoxy, isopropoxy, n-butoxy, isobutoxy, n-pentyloxy, isopentyloxy, n-hexyloxy, isohexyloxy and 3,3-dimethyl butoxy;
preferably, the halogen is selected from fluorine, chlorine, bromine and iodine.

5. The phosphine-phenol transition metal complex as claimed in claim 2, **characterized in that**, it is selected from a group consisting of the following complexes,
complex 1: complex of formula (II), wherein M is Ti, R₁₁-R₁₅ are H, X is Cl, n=1;
complex 2: complex of formula (II), wherein M is Ti, R₁₁-R₁₅ are H, X is Cl, n=2;
complex 3: complex of formula (II), wherein M is Ti, R₁₁-R₁₅ are H, X is methyl, n=1;
complex 4: complex of formula (II), wherein M is Ti, R₁₁-R₁₅ are H, X is -CH₂C₆H₅, n=2;
complex 5: complex of formula (II), wherein M is Ti, R₁₁, R₁₂, R₁₄ and R₁₅ are H, R₁₃ is -CF₃, X is Cl, n=1;
complex 6: complex of formula (II), wherein M is Ti, R₁₁, R₁₂, R₁₄ and R₁₅ are H, R₁₃ is -CF₃, X is Cl, n=2;
complex 7: complex of formula (II), wherein M is Ti, R₁₁, R₁₂, R₁₄ and R₁₅ are H, R₁₃ is -CF₃, X is methyl, n=2;
complex 8: complex of formula (II), wherein M is Ti, R₁₁, R₁₂, R₁₄ and R₁₅ are H, R₁₃ is -CF₃, X is -CH₂C₆H₅, n=2;
complex 9: complex of formula (II), wherein M is Ti, R₁₂-R₁₅ are H, R₁₁ is methoxy, X is Cl, n=1;
complex 10: complex of formula (II), wherein M is Ti, R₁₂-R₁₅ are H, R₁₁ is methoxy, X is Cl, n=2;
complex 11: complex of formula (II), wherein M is Ti, R₁₂-R₁₅ are H, R₁₁ is methoxy, X is methyl, n=2;
complex 12: complex of formula (II), wherein M is Ti, R₁₂-R₁₅ are H, R₁₁ is methoxy, X is - CH₂C₆H₅, n=2;
complex 13: complex of formula (II), wherein M is Ti, R₁₁, R₁₂, R₁₄ and R₁₅ are H, R₁₃ is methoxy, X is Cl, n=1;
complex 14: complex of formula (II), wherein M is Ti, R₁₁, R₁₂, R₁₄ and R₁₅ are H, R₁₃ is methoxy, X is Cl, n=2;
complex 15: complex of formula (II), wherein M is Ti, R₁₁, R₁₂, R₁₄ and R₁₅ are H, R₁₃ is methoxy, X is methyl, n=2;
complex 16: complex of formula (II), wherein M is Ti, R₁₁, R₁₂, R₁₄ and R₁₅ are H, R₁₃ is methoxy, X is -CH₂C₆H₅, n=2;
complex 17: complex of formula (II), wherein M is Ti, R₁₁, R₁₃ and R₁₅ are H, R₁₂ and R₁₄ are - CF₃, X is Cl, n=1;
complex 18: complex of formula (II), wherein M is Ti, R₁₁, R₁₃ and R₁₅ are H, R₁₂ and R₁₄ are - CF₃, X is Cl, n=2;
complex 19: complex of formula (II), wherein M is Ti, R₁₁, R₁₃ and R₁₅ are H, R₁₂ and R₁₄ are - CF₃, X is methyl, n=2;
complex 20: complex of formula (II), wherein M is Ti, R₁₁, R₁₃ and R₁₅ are H, R₁₂ and R₁₄ are - CF₃, X is -CH₂C₆H₅, n=2;
complex 21: complex of formula (II), wherein M is Ti, R₁₁, R₁₃ and R₁₅ are H, R₁₂ and R₁₄ are methyl, X is Cl, n=1;
complex 22: complex of formula (II), wherein M is Ti, R₁₁, R₁₃ and R₁₅ are H, R₁₂ and R₁₄ are methyl, X is Cl, n=2;
complex 23: complex of formula (II), wherein M is Ti, R₁₁, R₁₃ and R₁₅ are H, R₁₂ and R₁₄ are methyl, X is methyl, n=2;
complex 24: complex of formula (II), wherein M is Ti, R₁₁, R₁₃ and R₁₅ are H, R₁₂ and R₁₄ are methyl, X is -CH₂C₆H₅, n=2;
complex 25: complex of formula (II), wherein M is Zr, R₁₁-R₁₅ are H, X is Cl, n=2;
complex 26: complex of formula (II), wherein M is Zr, R₁₁-R₁₅ are H, X is methyl, n=2;
complex 27: complex of formula (II), wherein M is Zr, R₁₁-R₁₅ are H, X is -CH₂C₆H₅, n=2;
complex 28: complex of formula (II), wherein M is Zr, R₁₁, R₁₂, R₁₄ and R₁₅ are H, R₁₃ is -CF₃, X is Cl, n=2;
complex 29: complex of formula (II), wherein M is Zr, R₁₁, R₁₂, R₁₄ and R₁₅ are H, R₁₃ is -CF₃, X is methyl, n=2;
complex 30: complex of formula (II), wherein M is Zr, R₁₁, R₁₂, R₁₄ and R₁₅ are H, R₁₃ is -CF₃, X is -CH₂C₆H₅, n=2;
complex 31: complex of formula (II), wherein M is Zr, R₁₂-R₁₅ are H, R₁₁ is methoxy, X is Cl, n=2;
complex 32: complex of formula (II), wherein M is Zr, R₁₂-R₁₅ are H, R₁₁ is methoxy, X is methyl, n=2;
complex 33: complex of formula (II), wherein M is Zr, R₁₂-R₁₅ are H, R₁₁ is methoxy, X is - CH₂C₆H₅, n=2;
complex 34: complex of formula (II), wherein M is Zr, R₁₁, R₁₂, R₁₄ and R₁₅ are H, R₁₃ is methoxy, X is Cl, n=2;
complex 35: complex of formula (II), wherein M is Zr, R₁₁, R₁₂, R₁₄ and R₁₅ are H, R₁₃ is methoxy, X is methyl, n=2;
complex 36: complex of formula (II), wherein M is Zr, R₁₁, R₁₂, R₁₄ and R₁₅ are H, R₁₃ is methoxy, X is -CH₂C₆H₅, n=2;
complex 37: complex of formula (II), wherein M is Zr, R₁₁, R₁₃ and R₁₅ are H, R₁₂ and R₁₄ are - CF₃, X is Cl, n=2;
complex 38: complex of formula (II), wherein M is Zr, R₁₁, R₁₃ and R₁₅ are H, R₁₂ and R₁₄ are - CF₃, X is methyl, n=2;
complex 39: complex of formula (II), wherein M is Zr, R₁₁, R₁₃ and R₁₅ are H, R₁₂ and R₁₄ are - CF₃, X is -CH₂C₆H₅, n=2;
complex 40: complex of formula (II), wherein M is Zr, R₁₁, R₁₃ and R₁₅ are H, R₁₂ and R₁₄ are methyl, X is Cl, n=2;
complex 41: complex of formula (II), wherein M is Zr, R₁₁, R₁₃ and R₁₅ are H, R₁₂ and R₁₄ are methyl, X is methyl, n=2;
complex 42: complex of formula (II), wherein M is Zr, R₁₁, R₁₃ and R₁₅ are H, R₁₂ and R₁₄ are methyl, X is -CH₂C₆H₅, n=2;
complex 43: complex of formula (II), wherein M is Hf, R₁₁-R₁₅ are H, X is Cl, n=2;
complex 44: complex of formula (II), wherein M is Hf, R₁₁-R₁₅ are H, X is methyl, n=2;
complex 45: complex of formula (II), wherein M is Hf, R₁₁-R₁₅ are H, X is -CH₂C₆H₅, n=2;
complex 46: complex of formula (II), wherein M is Hf, R₁₁, R₁₂, R₁₄ and R₁₅ are H, R₁₃ is -CF₃, X is Cl, n=2;
complex 47: complex of formula (II), wherein M is Hf, R₁₁, R₁₂, R₁₄ and R₁₅ are H, R₁₃ is methoxy, X is Cl, n=2;
complex 48: complex of formula (II), wherein M is Hf, R₁₁, R₁₃ and R₁₅ are H, R₁₂ and R₁₄ are - CF₃, X is Cl, n=2;
complex 49: complex of formula (II), wherein M is Hf, R₁₁, R₁₃ and R₁₅ are H, R₁₂ and R₁₄ are methyl, X is Cl, n=2;
complex 50: complex of formula (II), wherein M is Hf, R₁₁, R₁₃ and R₁₅ are H, R₁₂ and R₁₄ are - CF₃, X is -CH₂C₆H₅, n=2;
complex 51: complex of formula (II), wherein M is Hf, R₁₁, R₁₂, R₁₄ and R₁₅ are H, R₁₃ is -CF₃, X is -CH₂C₆H₅, n=2;
complex 52: complex of formula (II), wherein M is Hf, R₁₁, R₁₂, R₁₄ and R₁₅ are H, R₁₃ is methoxy, X is -CH₂C₆H₅, n=2;
complex 53: complex of formula (II), wherein M is Hf, R₁₁, R₁₃ and R₁₅ are H, R₁₂ and R₁₄ are methyl, X is -CH₂C₆H₅, n=2;
complex 54: complex of formula (II), wherein M is Hf, R₁₁, R₁₃ and R₁₅ are H, R₁₂ and R₁₄ are - CF₃, X is -CH₂C₆H₅, n=2.

6. A method for preparing the phosphine-phenol transition metal complex as claimed in any of claims 1-5, **characterized in that** the method comprises:
(1) reacting a compound of formula (III) with a compound of formula (IV), to obtain a ligand of formula (V);
(2) reacting the ligand with a hydrogen abstracting agent, and then reacting with an M metal compound, wherein, the transition metal M in the M metal compound is selected from Group IVB metals; 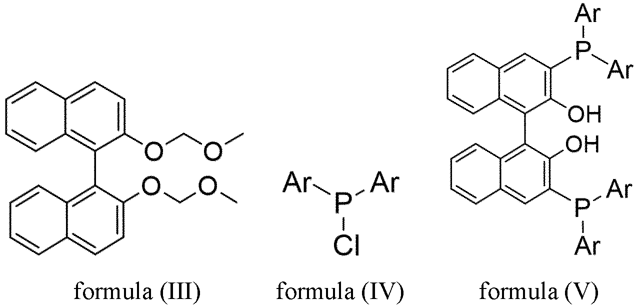 wherein, the definition of Ar is the same as that in claim 1.

7. The method as claimed in claim 6, **characterized in that**, the M metal compound is selected from at least one of titanium tetrachloride, bis(tetrahydrofuran) titanium tetrachloride, tri(tetrahydrofuran) titanium trichloride, zirconium tetrachloride, bis(tetrahydrofuran) zirconium tetrachloride, hafnium tetrachloride, and bis(tetrahydrofuran) hafnium tetrachloride.

8. The method as claimed in claim 6, **characterized in that**, the hydrogen abstracting agent is selected from at least one of NaH, KH, n-butyl lithium and methyl lithium.

9. Use of the phosphine-phenol transition metal complex as claimed in any of claims 1-5 in olefin polymerization.

10. An olefin polymerization catalyst, **characterized in that** it comprises the phosphine-phenol transition metal complex as claimed in any of claims 1-5 and a cocatalyst.

11. The olefin polymerization catalyst as claimed in claim 10, **characterized in that**, the cocatalyst is an organoaluminum compound and/or an organoboron compound;
preferably, the organoaluminum compound is selected from one or more of alkylaluminoxane, alkyl aluminum and alkyl aluminum halide;
preferably, the organoboron compound is selected from one or more of organoboron and organoborate.

12. An olefin polymerization method, **characterized in that** it comprises carrying out olefin polymerization reaction in the presence of the olefin polymerization catalyst as claimed in claim 10 or 11;
preferably, the olefin polymerization reaction has a temperature of -78°C~200°C, preferably -20°C~150°C, and a pressure of 0.01~10MPa, preferably 0.01~5MPa.
